# EUROPEAN PATENT SPECIFICATION

(11) **EP 1 792 561 B1**
(45) Date of publication and mention of the grant of the patent: **11.06.2014**
(21) Application number: 05425854.6
(22) Date of filing: 30.11.2005
(51) Int. Cl.: A61B 1/00, A61B 1/31, A61M 25/01

(54) **Self-propelled endoscopic device**
Selbstfahrende endoskopische Vorrichtung
Dispositif d'endoscope automoteur

(43) Date of publication of application: 06.06.2007
(73) Proprietor: Era Endoscopy S.r.l., 56037 Peccioli (Pisa) (IT)
(72) Inventor: Arena, Alberto, 56021 Marciana di Cascina (Pisa) (IT); Dario, Paolo, 57100 Livorno (IT); Gorini, Samuele, 56020 Montecalvoli (Pisa) (IT); Menciassi, Arianna, 56020 Pontedera (Pisa) (IT); Pernorio, Giuseppe, 56100 Pisa (IT)
(74) Representative: Brazzini, Silvia

(56) References cited:
- EP-A2- 0 838 200
- WO-A-02/068035
- WO-A-03/053505
- WO-A-2005/094665
- US-A- 5 398 670
- US-A- 5 662 587
- US-A1- 2005 070 949

## Description

The present invention relates to an improvement to an endoscopic device for locomotion through a tubular body cavity and particularly, but not exclusively, through the gastrointestinal tract, capable of migrating in a pre-established direction by so-called inchworm motion.

Endoscopic devices for surgical or diagnostic procedures are already known, which are operated by the surgeon who directly imparts to the device its forward motion through the patient's body. These devices are generally associated with surgical and/or diagnostic instruments needed to complete various procedures, e.g. micro-arms, micro-cameras and/or laser emitters.

Endoscopic devices of this type, but capable of autonomous or semi-autonomous locomotion through the body cavity of a patient are described, for instance, in US-A-5398670, US-A-5906591,EP0838200 and WO02/068035. The endoscopic device described in these documents substantially consists of a tubular body of variable length with two, front and rear end portions, complete with anchoring means that enable said front end portion or rear end portions to become temporarily and alternately attached to the wall of the body cavity, thereby enabling the forward motion of the device.

In particular, the variable-length tubular body of the endoscopic device described in the above-mentioned documents is in the form of a bellows-shaped tube and is consequently capable of being extended or contracted by means of air injected therein or aspirated therefrom. In the above-mentioned patent application PCT n. WO02/068035, the device is anchored to the wall of the body cavity by clamping means associated with the front and rear end portions of the device and selectively enabled by an external control unit in synchronism with the successive extensions and contractions of the bellows-shaped tubular body. The aforementioned clamping means are enabled by pneumatic actuating means that, in the preferred embodiment of the invention, also consist of bellows-shaped members.

When it is extended, a positive pressure is created inside the bellows by means of compressed air, thereby obtaining an elongation proportional to the pressure therein, while the bellows are contracted by progressively reducing the pressure inside the bellows, until some degree of vacuum is created.

Another endoscopic device in which the inchworm locomotion is performed through bellows type extensor modules is disclosed in US 5662587. In this case the extensor module is reinforced with a metal wire embedded in the wall of the bellows.

Although it has the considerable flexibility needed to negotiate the narrow intestinal loops without causing pain, the device according to the above-mentioned PCT patent application has several functional drawbacks due to its relative extendibility and friction between its outer surface and the walls of the body cavity, which have a negative effect on the device's efficiency of locomotion. Because the intestinal walls adhere partially or totally to the outer surface of the bellows, the intestinal tissue may be trapped between the folds of the bellows during the contraction stage. Though it does no damage the mucosa, this interferes with the progress of the device through the intestines. Moreover, given the elasticity of the mesentery and intestinal tissues, any friction between the surface of the tubular bellows-shaped body and the walls of the body cavity will stretch the tissue and mesentery instead of making the device slide along the walls, thus preventing any forward motion of the device relative to the intestinal walls during the extension stage, then allowing the tissues of the tubular cavity and the mesentery to return to their original position (with a so-called "accordion effect") during the contraction stage.

It should be noted, moreover, that the considerable thickness of the bellows-shaped tubular body (in terms of the difference between the maximum radius and the minimum radius of the contracted and extended bellows) results in a significant reduction in the space actually available inside it, making difficult the passage of the compressed air tubes needed for the displacement of the device and making it necessary to use smaller-diameter tubes, with a consequent increase in the pressure drop and reduction in the device's speed of locomotion.

The object of the present invention is to provide an endoscopic device of the type described in the international patent application WO02/068035, preserving the same degree of flexibility without presenting the above-described functional drawbacks.

A particular object of the present invention is to provide an endoscopic device of the above-mentioned type wherein the tubular body has a considerable capacity for extension and a low friction coefficient so as to avoid any entrainment of the tissues forming the wall of the body cavity and thus ensure an effective locomotion of the device.

Another particular object of the present invention is to provide an endoscopic device of the aforementioned type with a significantly more spacious interior, while maintaining the same outer diameter, than is available in endoscopic devices of known type with bellows-shaped bodies, thereby enabling said interior to be used more efficiently for the passage of the service tubes.

A further object of the present invention is to provide an endoscopic device of the above-mentioned type, wherein its tubular body, or the actuators of the anchoring means provided on its front and rear end portions, will contract without giving rise to any circumferential surface folds in which the tissue of the wall forming the body cavity through which the endoscopic device is advancing might become trapped.

These objects are achieved by the improved endoscopic device according to the present invention, in which its tubular body is made of an elastic material and incorporates a reinforcement structure distributed along its length that is substantially rigid in the radial direction and yielding in the axial direction. In the preferred embodiments, said reinforcement consists either of a plurality of substantially rigid rings, or of at least one coaxial spring, or preferably a pair of springs wound crosswise to one another, incorporated within its thickness.

The characteristics and advantages of the improved endoscopic device according to the present invention will be more apparent from the following description of a preferred embodiment, given as a non-limiting example with reference to the attached drawings, wherein:
- figure 1 shows schematically the improved endoscopic device according to the present invention;
- figure 2 is an enlarged cross-sectional view of a first embodiment of the central tubular body of the endoscopic device according to the present invention;
- figure 3 is a perspective view of a second embodiment of the central tubular body of the endoscopic device according to the present invention;
- figure 4 is a longitudinal cross-sectional view of the central tubular body shown of figure 3;
- figures 5 and 6 schematically illustrate one end portion of the endoscopic device according to the present invention, with the anchoring means respectively in the open and closed positions.

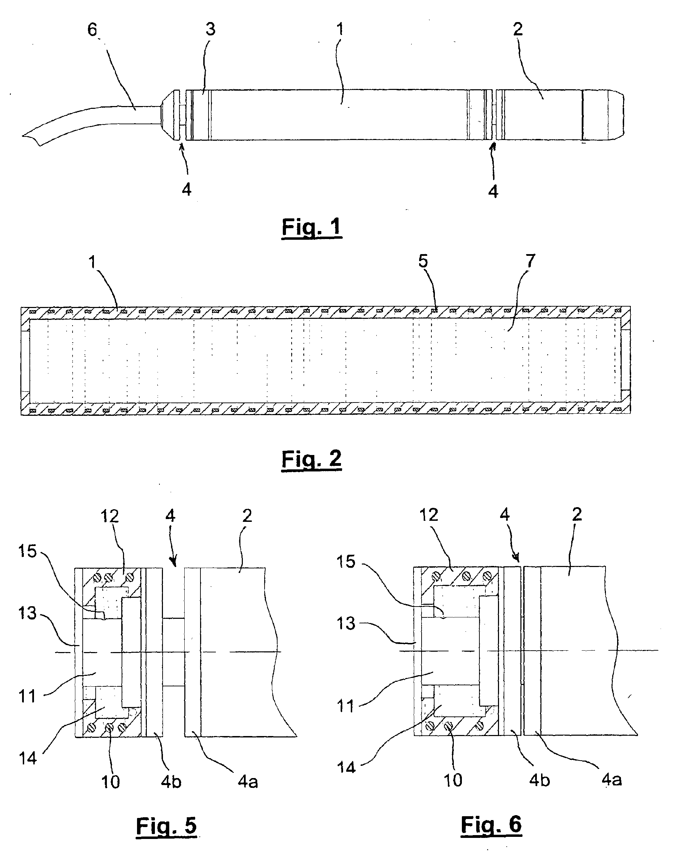

With reference to figure 1, the endoscopic device according to the present invention comprises of a tubular body 1 extending between two end portions, called the front 2 and rear 3 end portions respectively, where the terms front and rear refer to the device's direction of locomotion through the body cavity, indicated by the arrow F. Clearly, the device is movable in both forward and reverse direction within the body cavity.

The front 2 and rear 3 end portions of the device include anchoring means 4, specifically of the clamping type, by means of which the device temporarily and alternately becomes attached to the wall of the body cavity to enable its locomotion in a known manner.

According to the present invention, the tubular body 1 is made of a low-hardness elastomeric material, e.g. Shore A 10 silicone. The tubular body 1 has a structural reinforcement comprising, in a first embodiment of the invention illustrated in figure 2, a plurality of rings 5 made of a rigid material, e.g. Shore A 80 silicone.

The rear end portion 3 is connected to an external control unit by means of a hose 6, which houses the service tubes, including the tubing needed to deliver compressed air inside the tubular body 1, or to create a negative pressure therein, thereby inducing the extension or retraction of the tubular body 1 that is necessary for the known so-called inchworm type of locomotion.

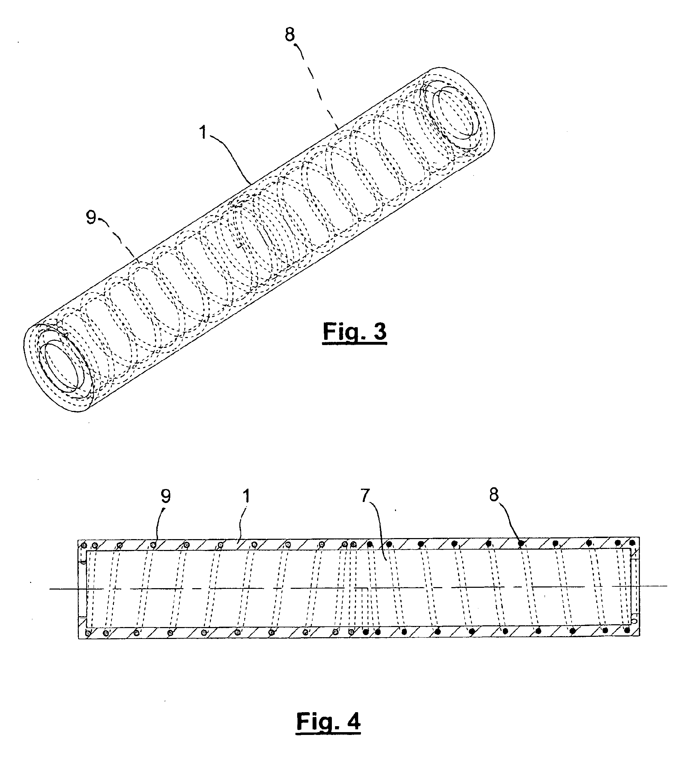

In an alternative embodiment of the invention, illustrated in figures 3 and 4, the silicone tubular body 1 is reinforced with a pair of helical springs 8 and 9 made of a rigid material, e.g. steel, wound crosswise to one another.

In both cases, the presence of the plurality of rings or of the two coaxial springs prevents any radial dilation or collapse of the tubular body 1, while still allowing its extension and retraction in the axial direction according to changes in the internal pressure conditions.

In the case involving a pair of helical springs, 8 and 9, the arrangement of the springs with the windings lying crosswise to one another prevents any related rotation of the device's end portions 2 and 3 (which would induce a continuous rotation of the image transmitted by a TV camera installed in the front end portion 2) or any twisting of the hose 6 extending from the rear end portion 3. The fact that the two springs 8 and 9 are wound crosswise to one another also facilitates the sliding of the tissue of the body cavity over the outer surface of the tubular body 1.

Silicone tubing reinforced with rings 5, or a helical spring 10, as illustrated in figures 5 and 6, can also be used for the actuating device that controls the opening and closing of the anchoring means 4 installed at the front 2 and rear 3 end portions of the endoscopic device. As illustrated in the above-mentioned figures, the anchoring means 4 comprise a pair of circular jaws 4a and 4b, the former of which is fixed, while the latter is movable with respect to the former. In particular, the movable jaw 4b is slidingly mounted on a tubular, cylindrical member 11 extending at right angles from a connection flange 13 by means of which the end portion 2 is axially connected to the tubular body 1. The movable jaw 4b is also connected to the flange 13 by means of a silicone tube 12 with a helical spring 10 (in the case illustrated) incorporated within its thickness. The tube 12 delimits a chamber 14 into which compressed air can be delivered or a negative pressure can be created through a hole 15 in the tubular member 11. The creation of a positive or negative pressure in the chamber 14 results in the extension or contraction of the tube 12, which in turn makes the movable jaw 4b slide one way or the other and, as a consequence, respectively close or open the anchoring means 4.

The improvement according to the present invention, applied both to the tubular body and to the pneumatic actuators of the anchoring means, ensures an efficient locomotion of the endoscopic device, overcoming all the above-mentioned drawbacks of the known devices. In fact, the outer surface of the device remains smooth and slippery both in extension and in contraction, without any folds formation in which the tissue of the body cavity wall could be trapped. Moreover, the absence of the typical folds of a bellows design leads to an increase in the ratio of the internal to the external diameter of the device, affording a significantly larger internal volume for the same external diameter, which facilitates the passage of the service tubes. Finally, the highly-extendable tubular body 1 combines with a low friction coefficient to prevent any stretching of the body cavity wall tissues, which would slow down the forward displacement of the device reducing its locomotion efficiency.

## Claims

1. Endoscopic device for self-propelled locomotion through a body cavity in a pre-established direction of displacement, comprising a tubular body (1) extending between two end portions, respectively front (2) and rear (3) end portions, comprising anchoring means (4) suitable for temporarily and alternately attaching said end portions to the wall of the body cavity in synchronism with corresponding axial extensions and contractions of said tubular body (1), and pneumatic means (12) for actuating said anchoring means, said tubular body (1) incorporating a reinforcement structure (5,8,9) distributed along its length that is substantially rigid in the radial direction and yielding in the axial direction, **characterized in that** said tubular body (1) is made of an elastic material and has an outer surface which remains smooth and slippery both in extension and contraction, without formation of folds, thereby avoiding the tissue of the said body cavity wall to be trapped and affording a larger internal volume of said device for a same external diameter, and said tubular body (1) has a low friction coefficient thereby preventing any stretching of said body cavity wall tissues.

2. Endoscopic device as set forth in claim 1, wherein said pneumatic means for actuating said anchoring means (4) comprise a tubular member (12) made of a flexible and elastic material also incorporating said reinforcement structure, connected axially to a movable member (4b) of said anchoring means and to said tubular body (1) and capable of extending and contracting cyclically in response to positive and negative pressures being created in its internal chamber (14), thus causing the closure and, respectively, the opening of said anchoring means (4).

3. Endoscopic device as set forth in claim 2, wherein said anchoring means (4) are of the clamp type, and are formed by a fixed jaw (4a) and a mobile jaw (4b), to which said tubular member (12) is connected.

4. Endoscopic device as set forth in any of the previous claims, wherein said reinforcement structure comprises a plurality of substantially rigid rings (5) embedded coaxially within the thickness of said tubular body (1) and said tubular member (12).

5. Endoscopic device as set forth in claims 1, 2 or 3, wherein said reinforcement structure comprises at least one helical spring (8,9,10) coaxially incorporated within the thickness of said tubular body (1) and said tubular member (12).

6. Endoscopic device as set forth in claim 5, wherein the structure for reinforcing said tubular body (1) comprises a pair of coaxial helical springs (8,9) wound crosswise to one another.

7. Endoscopic device as set forth in claim 2, wherein said elastic and flexible material is low-hardness silicone.

8. Endoscopic device as set forth in claim 4, wherein said rings (5) are made of silicone Shore A 80.

## Patentansprüche

1. Endoskopische Vorrichtung zur selbstfahrenden Fortbewegung durch einen Körperhohlraum in einer voreingestellten Verstellungsrichtung, enthaltend einen rohrartigen Körper (1), der sich zwischen zwei Endteilen erstreckt, die entsprechend vordere (2) und hintere Endteile (3) sind, die Ankereinrichtungen (4), die geeignet sind, um die Endteile temporär und abwechselnd an der Wand des Körperhohlraums in Synchronisation mit entsprechenden axialen Ausdehnungen und Zusammenziehungen des rohrartigen Körpers (1) zu befestigen, und Pneumatikeinrichtungen (12) enthalten, um die Ankereinrichtungen zu betätigen, wobei der rohrartigen Körper (1) eine entlang seiner Länge verteilte Verstärkungsstruktur (5, 8, 9) enthält, die im Wesentlichen in der Radialrichtung steif und nachgiebig in der Axialrichtung ist, **dadurch gekennzeichnet, dass** der rohrartige Körper (1) aus einem elastischen Material besteht und eine Außenoberfläche hat, die sowohl bei der Ausdehnung, als auch bei der Zusammenziehung glatt und rutschig bleibt, ohne Falten zu bilden, wodurch vermieden wird, dass das Gewebe der Wand des Körperhohlraums eingeklemmt wird, und ein größeres internes Volumen der Vorrichtung beim selben äußeren Durchmesser geboten wird, und der rohrartige Körper (1) einen niedrigen Reibungskoeffizienten hat, wodurch jegliches Dehnen der Gewebe der Wand des Körperhohlraums verhindert wird.

2. Endoskopische Vorrichtung nach Anspruch 1, wobei die Pneumatikeinrichtungen zum Betätigen der Ankereinrichtungen (4) ein rohrartiges, aus einem flexiblen und elastischen Material, das auch die Verstärkungsstruktur enthält, bestehendes Element (12) enthalten, das axial mit einem beweglichen Element (4b) der Ankereinrichtungen und mit dem rohrartigen Körper (1) verbunden ist und geeignet ist, sich in Abhängigkeit von positiven und negativen Drücken zyklisch auszudehnen und zusammenzuziehen, die in seiner internen Kammer (14) erzeugt werden, womit das Schließen und entsprechend Öffnen der Ankereinrichtungen (4) veranlasst wird.

3. Endoskopische Vorrichtung nach Anspruch 2, wobei die Ankereinrichtungen (4) vom Klammertyp sind und durch eine feststehende Backe (4a) und eine bewegliche Backe (4b) gebildet sind, mit der das rohrartige Element (12) verbunden ist.

4. Endoskopische Vorrichtung nach einem der vorhergehenden Ansprüche, wobei die Verstärkungsstruktur eine Mehrzahl von im Wesentlichen steifen Ringen (5) enthält, die koaxial innerhalb der Dicke des rohrartigen Körpers (1) und des rohrartigen Elements (12) eingebettet sind.

5. Endoskopische Vorrichtung nach einem der Ansprüche 1, 2 oder 3, wobei die Verstärkungsstruktur wenigstens eine spiralförmige Feder (8, 9, 10) enthält, die koaxial innerhalb der Dicke des rohrartigen Körpers (1) und des rohrartigen Elements (12) inkorporiert ist.

6. Endoskopische Vorrichtung nach Anspruch 5, wobei die Struktur zum Verstärken des rohrartigen Körpers (1) ein Paar von koaxialen spiralförmige Federn (8, 9) enthält, die überkreuz zueinander gewickelt sind.

7. Endoskopische Vorrichtung nach Anspruch 2, wobei das elastische und flexible Material Silikon geringer Härte ist.

8. Endoskopische Vorrichtung nach Anspruch 4, wobei die Ringe (5) aus Silikon Shore A 80 bestehen.

## Revendications

1. Dispositif endoscopique pour un déplacement autopropulsé dans une cavité corporelle suivant une direction de déplacement préétablie, comprenant un corps tubulaire (1) s'étendant entre deux portions d'extrémités, respectivement une portion frontale (2) et une portion arrière (3), comprenant des moyens d'attache (4) appropriés pour attacher temporairement et alternativement lesdites portions d'extrémités à la paroi de la cavité corporelle en synchronisme avec des dilatations et des contractions axiales correspondantes du dit corps tubulaire (1), et des moyens pneumatiques (12) pour actionner lesdits moyens d'attache, ledit le corps tubulaire (1) incorporant une structure de renfort (5,8,9) répartie sur sa longueur qui est sensiblement rigide en direction radiale et flexible en direction axiale, **caractérisé en ce que** ledit corps tubulaire (1) est réalisé en un matériau élastique et présente une surface extérieure qui reste lisse et glissante à la fois en dilatation et en contraction, sans formation de plis, évitant ainsi au tissu de ladite paroi de la cavité corporelle d'être immobilisé et d'occuper un plus grand volume à l'intérieur du dit dispositif pour un même diamètre extérieur, et que ledit corps tubulaire (1) présente un faible coefficient de friction, empêchant ainsi tout étirement des dits tissus de la paroi de la cavité du corporelle.

2. Dispositif endoscopique comme défini dans la revendication 1, où lesdits moyens pneumatiques pour actionner lesdits moyens d'attache (4) comportent un élément tubulaire (12) réalisé dans un matériau flexible et élastique incorporant aussi ladite structure de renfort, relié axialement à un élément mobile (4b) des dits moyens d'attache et au dit corps tubulaire (1) et apte à se dilater et se contracter cycliquement en réponse à des pressions positives et négatives créées dans sa chambre intérieure (14), entraînant de ce fait la fermeture et, respectivement, l'ouverture des dits moyens d'attache (4).

3. Dispositif endoscopique comme défini dans la revendication 2, où lesdits moyens d'attache (4) sont du type pince, et sont constitués d'une mâchoire fixe (4a) et d'une mâchoire mobile (4b) à laquelle est relié ledit élément tubulaire (12).

4. Dispositif endoscopique comme défini dans l'une quelconque des revendications précédentes, où ladite structure de renfort comporte une pluralité des anneaux sensiblement rigides (5) noyés coaxialement dans l'épaisseur du dit corps tubulaire (1) et du dit élément tubulaire (12).

5. Dispositif endoscopique comme défini dans l'une des revendications 1 à 3, où ladite structure de renfort comporte au moins un ressort hélicoïdal (8, 9,10) incorporé coaxialement dans l'épaisseur du dit corps tubulaire (1) et du dit élément tubulaire (12).

6. Dispositif endoscopique comme défini dans la revendication 5, où la structure pour renforcer ledit corps tubulaire (1) comporte une paire de ressorts hélicoïdaux coaxiaux (8, 9) enroulés en sens inverse l'un par rapport à l'autre.

7. Dispositif endoscopique comme défini dans la revendication 2, où ledit matériau élastique et flexible est un silicone faible-dureté.

8. Dispositif endoscopique comme défini dans la revendication 4, où lesdits anneaux (5) sont faits en silicone Shore A 80.
